# EUROPEAN PATENT APPLICATION

(11) **EP 1 911 750 A1**
(43) Date of publication of application: **16.04.2008**
(21) Application number: 06781847.6
(22) Date of filing: 21.07.2006
(51) Int. Cl.: C07D 277/20, C07D 277/40, C07D 277/46

(54) **AGENT FOR IMPROVEMENT IN BOUNCE/BODY OF HAIR, AND HAIR COSMETIC**

(30) Priority: 29.07.2005 JP 2005220504; 29.08.2005 JP 2005247571
(71) Applicant: Shiseido Company, Limited, Chuo-ku Tokyo 104-8010 (JP)
(72) Inventor: SUETSUGU, Masaru, c/o SHISEIDO RESEARCH CENTER, Yokohama-shi, Kanagwa 2248558 (JP); FUKUNISHI, Hirotada, c/o SHISEIDO RESEARCH CENTER, Yokohama-shi, Kanagawa 2248558 (JP); IINO, Masato, c/o SHISEIDO RESEARCH CENTER, Yokohama-shi, Kanagwa 2248558 (JP); YAMAKI, Satoshi, c/o SHISEIDO RESEARCH CENTER, Yokohama-shi, Kanagawa 2248558 (JP); SOMA, Tsutomu, c/o SHISEIDO RESEARCH CENTER, Yokohama-shi, Kanagawa 2248558 (JP)
(74) Representative: Henkel, Feiler & Hänzel
(86) International application number: PCT/JP2006/314936
(87) International publication number: WO 2007/013572

(57) **Abstract**

A hair resiliency and body improver comprising as the active ingredient thereof one or more types of compounds selected from the group consisting of 2-aminothiazole derivatives represented by the following general formula (1) or 3-aminoisoxazole derivatives represented by the following general formula (2), and pharmaceutically acceptable salts thereof: 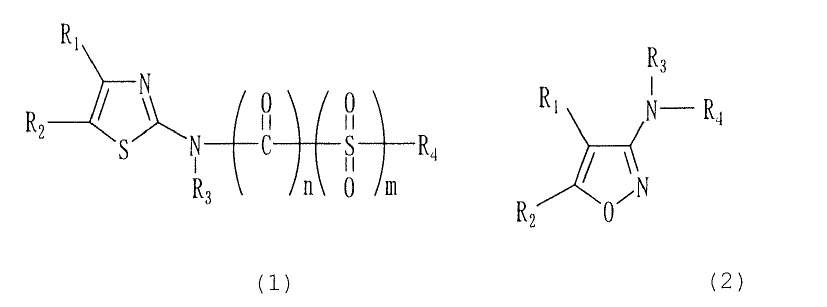 (wherein, R₁, R₂, R₃, R₄, n and m are as defined in the description).

SEQUENCE LISTING
<110> Shiseido CO. LTD.
<120> Hair Fitness/Body Improving Agents and Hair Cosmetics
<130> S745

<150> JP2005-220504
<151> 2005-07-29

<150> JP2005-247571
<151> 2005-08-29

<160> 8

<210> 1
<211> 498
<212> DNA
<213> Homo Sapiens

<220>
<222> (1)...(498)

<220>
<223> KAP5.1

<300>
<308> AP000867

<400> 1
atgggctgct gtggctgttc cgaaggctgt ggctccggct gtgggggctg tggctccggc
tgtgggggct gtggctctgg ctgtggggga tgtggctcca gctgctgtgt gcccgtctgc
tgctgcaagc ccgtgtgctg ctgtgtgcca gcctgttcct gctccagctg tggctcctgt
gggggctcca agggaggctg tggctcctgt gggggctcca aggggggctg tggctcttgt
gggggttcta aggggggctg tggttcttgt ggctgctccc agtgcagctg ctataagccc
tgctgctgct cctcaggctg tgggtcatcc tgctgccagt ccagctgctg taagccctgc
tgctgccagt ccagctgctg taagccctgc tgctgttcct caggctgtgg gtcatcctgc

tgccagtcca gttgctgcaa tccctgctgc tcccagtcta gctgctgtgt ccccgtgtgc
tgccagtgta agatctga

<210> 2
<211> 564
<212> DNA
<213> Homo Sapiens

<220>
<222> (1)... (564)

<220>
<223> KAP5.2

<300>
<308> AP000867

<400> 2
atgggctgct gtggctgctc tggaggctgt ggctccggct gtgggggctg cggctctggc
tgtgggggat gtggctctag ctgctgtgtg cccatctgct gctgcaagcc cgtgtgctgc
tgtgtgccag cctgttcctg ctccagctgt ggctcctgtg ggggctccaa ggggggccgt
ggctcctgtg ggggctccaa gggggactgt ggctcctgtg ggggctccaa gggaggctgt
ggttcttgtg gctgctccca gtgcagctgc tataagccct gctgttgctc ctcaggctgt
gggtcatcct gctgccagtc cagctgctgc aaaccctgct gttcccagtc cagctgttgt
aagccctgca gctgctcttc aggctgtggg tcatcctgct gccagtccag ctgctgcaag
ccctgctgtt cccagtccag ctgctgtaag ccctgctgct gctcttcagg ctgtgggtca
tcctgctgcc agtccagctg ctgcaagccc tgctgttccc agtccagctg ctgtgtccca
atttgctgcc agtgcaagat ctga

<210> 3
<211> 510
<212> DNA
<213> Homo Sapiens

<220>
<222> (1)... (510)

<220>
<223> KAP5.3

<300>
<308> AP000867

<400> 3
atgggctgct gtggctgctc cggaggctgt ggctccagct gtggaggctg tgactccagc
tgtgggagct gtggctctgg ctgcaggggc tgtggcccca gctgctgtgc acccgtctac
tgctgcaagc ccgtgtgctg ctgtgttcca gcctgttcct gctctagctg tggcaagcgg
ggctgtggct cctgtggggg ctccaaggga ggctgtggtt cttgtggctg ctcccagtgc
agttgctgca agccctgctg ttgctcttca ggctgtgggt catcctgctg ccagtgcagc
tgctgcaagc cctactgctc ccagtgcagc tgctgtaagc cctgttgctc ctcctcgggt
cgtgggtcat cctgctgcca atccagctgc tgcaagccct gctgctcatc ctcaggctgt
gggtcatcct gctgccagtc cagctgctgc aagccctgct gctcccagtc cagatgctgt
gtccctgtgt gctaccagtg caagatctga


<210> 4
<211> 609
<212> DNA
<213> Homo Sapiens

<220>
<222> (1)... (609)

<220>
<223> KAP5.4

<300>
<308> AP000867

<400> 4
atgggctgct gtggctgctc cggaggctgt ggctccggct gtgggggttg tggctccggc
tgtgggggct gtggctccgg ctgtgggggc tatggctctg gctgtggggg ctgtggctcc

agctgctgtg tgcccgtctg ctgctgcaag cccgtgtgct gctgtgtgcc agcctgttcc
tgctccagct gtggctcctg tgggggctcc aagggggact gtggctcttg tgggggctcc
aaagggggct gtggttcctg tgggggctcc aaggggggct gtggctcctg tgggggctcc
aaggggggct gtggttcttg tgggggctcc aaggggggct gtggctcctg tgggggctcc
aaaggtggct gtggttcctg tgggggctcc aaggggggct gtggttcttg tggctgctcc
cagtgcaatt gctgtaagcc ctgctgctgc tcctcaggct gtggatcctg ctgccagtcc
agctgctgca atccctgctg ctgccagtcc agctgctgtg tccccgtgtg ctgccagtct
agctgctgca agccctgctg ctgtcagtcc agctgctgtg tccccgtgtg ctgccagtgt
aagatctga

<210> 5
<211> 471
<212> DNA
<213> Homo Sapiens

<220>
<222> (1)... (471)

<220>
<223> KAP5.5

<300>
<308> AP000867

<400> 5
atgggctgct gtggctgttc tggaggctgt ggctctggct gtgggggctg tggctccggc
agtgggggct gtggctctgg ctgtgggggc tgtggctcca gctgctgtgt gcccatttgc
tgctgcaagc ctgtgtgctg ctgtgtgcca gcctgttcct gctccagctg tggctcctgt
gggggctcca aggggggctg tggctcttgt gggagctcca agggtgggtg tggttcttgt
ggctgctccc agtccaactg ctgtaagccc tgctgctcct cctcaggctg tgggtcattc
tgctgccagt ccagctgctc caaaccctgc tgctgccaat ccagctgctg ccagtccagc
tgctgcaagc cctgctgctg ccaatccagc tgctgccagt ctagctgctt caagccctgc
tgctgccagt ccagctgctg tgtccccgtg tgctgccagt gtaagatctg a

<210> 6

<211> 43
<212> DNA
<213> Artificial Sequence

<220>
<223> NM-F1

<400> 6
tttgcggccg cacgcgtttt cagtaatgtg cccctcttat gct

<210> 7
<211> 26
<212> DNA
<213> Artificial Sequence

<220>
<223> R2

<400> 7
aggttgaagt gatgaggtca ggaaag

<210> 8
<211> 4159
<212> DNA
<213> Homo sapiens

<220>
<222> (1)... (4159)

<220>
<223> KAP5. 1

<300>
<308> AP000867. 5

<400> 8
tttcagtaat gtgcccctct tatgctctcc tcagggttta gcatgcatcc cttaaacaaa
gaaagtagcc tcatataagc aatggttttg tgactagagt gcctcagtcc attattgctg
ctataagata ataattgacc tgggtaattg ataaagaaca gaaatgtact ttctcagagt
cctgaaggct gggaagtcca agatcaaggt gctgatatct ggtgtgggcc ttcttactgt
gtcttcacat attggaaggc agaagggcaa gagagcatga atatgttgtc ctcacatggc
agaagaacag aaaggggtga atgccctctc tcaagtcctt cttggagcag cactaattca
ttccaagtac cttttgttag gccccacctc ccaacactct tgcattgggg atgaagtttc
taatacatga attttggggg acacatgcag accatggcat aaaggaacct ctctccccac
atggagtaca actgagcagg gaacccagct gagcagctga agtaagcagc ctaagaggag
tgaacactct gcctggggaa gagagaaaac aacagcagag agacttgtga gattttcaga
aataattgag taggggaaaa gattccccac aaacaacaag atgaggatag attattctta
tttggatagg aaaggaaagg atattgtctg ggtgcagtgg ctcacacctg caatcccagc
actttgggag gccaaggtag gaggatcact ggaggccagg agtttgagac cagccagagc
aacatggcaa ggccccatct ctacaaaaag tttaaaagtt agctgggtat ggtgatacat
gcctatattc ccagctacct aggaggccaa ggcgggaaga ttccttaagc ccagaagttt
gaaggtgcag taagctgtgg tcacgtcact gcaatccagc ccaggtgaca gaggaagact
tcatctaaaa aaaaaaaagt gatccccagt aatatagttt gcatgcttcc tccaaatctt
atgttggtgg ggcctgctgg gaggtgtttg gatcatgggt gtagattttt catgaatggt
ttagcaccat ctccttggtg atgaataagt tcgagcaaga tctggttgtt taaaaatatg
tggcactgac caggcatggt ggctcatgca tgtcatctca acactttggg aggctgaggt
gggcagatca cctgaggtca ggagttcgag accagcctga ccaacaggga gaaaccctgt
ctctactaaa aatacaaaat tagccaggtg tggtggtgca tgcctgtact cccagctatt
cgggaggctg aggcaggaga atcacttgaa cctgggaagc ggaggttgcg gtgagccgag
atcatgccat tgtactccag cctgagtaac aagagcaaaa ctccatctca aaaaaaaaat
gtgtggcctc tgtccctcca ccagctctct ctttctcagc tgtcaccatg tggcatgcct
gctcctgctt tgccttctac catgaataaa agctccctga gggcctcacc agaagctgag
cagatgtcgg tgccatgctt gtacaactag cagaaccagg agccaattaa accttttta
tttataaatt acccagcctc agatatttct ttatggcaac acaaaaaata cagaaaactg
taccaggaat ggtatgttgc tataaagtca cctgaaaatg tggaggtgac tttggaactg
ggtaatggcc agagattgga agatttttgg agggctcaga agaagacaga aagatgagga
aaaggtggga actcctttga gactggttaa atggttgtgt ccaaaataat taaagtgata
agaacaatga agtccagcct gatgaggtct cagatggaaa tgaggaactt actgaaaact
aaaacaaaga tcacttgtgt tacaccttgg cagagagctt gaccatattg tgcttcatgc
cctagggggc tctgaaaatt tctacttagg agtgatgact taggatatct agtggaagaa

atttctaagc agcaaacatt caagctgtgg cctgactgaa tctaacatcg tattctcaca
tgcaagagca cataaatgaa ttaaagttgg aatttatatt taaaagggaa tcaaagtgta
aaagtttgga aactttgcag catggccatg tggcagagaa tgaaaaagca ttatcaggag
aggaatccaa gcaggctata gagcaaccac ctgctagaga tattagcatg actcaaaaaa
caaacaaaaa aacccaagtg ctaatagcca acacaattgg aaaagacttc aaatgcattt
caaaagtgtt tgagacagcc cctctcatca ctgacctaga ggtctaggag gacaccatgg
tttcaggggc tcaccccagg ccctcactgc cctgcatagc cttggagcac tgctccccac
acataggcta ctccagctcc agcctcagct caaaagaccc caaatgtaac tctggcttca
gagggtgcaa gccataagcc ttggtagctc ccatgtggtg ttaagcctgt aggtgcgtag
aatacaagag tgaaggaggc ttgggatcct ctacctagat tgcagaggat ttatgagaaa
gcctgggcac tcaggcagaa gcctgctgca ggggcagagc ccgcacagag gatatatact
agggcagtgt agaggggaaa tgtggagtag gaggcccaca cagagtcccc actggggcat
tgcttaattg agctgtgggg aggaggacac catcctccag gccccagaat ggtagagcca
ctggcagctt acatcttgag catggagaag ctgcaggcac acaactccaa cccatgagag
cagctgcagg gtgcgaacct tgcaaagcca caagggtgga gttccccaag gccttaggag
cccacccctc actccagtgt accctggatg tgggactggg agtccaagga cattattttg
gagctttaag atttaatggc tgccctgctg gatttcagac atgcatgggg tctgtagccc
cttcctttgg gctgatttct cccttttgga atgggaatgt ttacccaatg cctccaccct
catgtatctt caaagtaaat aatgtgtttt gattttcaca agttcattgg tggaagggac
ttggacttgg gactttggac ttgatgttgg aatgagtcaa gacattgaga ggactgttgg
gaagagatga ttctattctg caatgtaagg acatgagatt tgaggggtca aggggataat
gatacagttg ggatgtcccc tccaaatctc ttgtcgaaat gtaatcccca gtgttgaagg
aggggcctgc aaggaggtgt ttaggtcatg ggggcggatc cctcatgact ggcttagcgc
catctccttg gtgatgagtg tgttcactca gatctggttg ttcgcaagtg tgtggcccct
cctcacccct tgctcccact ctcacgatgt ggtgagcctg ctcctgcttc accttccacc
atgaggaaaa gctccctgag ggcctgccca gaagctgagc agaggtgggt gccatgcttg
tgcagcctgc agaaccatga gccattctaa cctttccttt ataaattacc cagcttcaag
tatttcttta gagcaatgca aaactgagaa tggcatgagt aaaatataaa gaccatggat
aattttgaaa tctaaaaatg tgaaattaat attcctgggg gagtcaacac aggaaagatg
agagcacgtg ggcaaaggct gttgcccatt ggtcacatgt ggtcagcaga gagcaggtga
ccagcaccct ggagctttgc gggaagcacc agacagcctg gagctgaggt ttcaattgta
ggggccaaaa caaataggga aaaagacaaa tttagtaaac aaagaaagta gatgctcaac
tgagccagga ggaagtcact agcaaagagg aaaaaaaata gagctctgga aaaatagcgt
aaacagtcac aaggaagcga ccctgtattg tgcctgacct ctggacaggt atataaagag
cccgggctca gggggctcca cacctgcacc tccctctcac ctgctcctct acctgctcca
ccctcaatcc accagaacc

## Description

### TECHNICAL FIELD

The present invention relates to a drug for improving the resiliency and body of hair and to a hair cosmetic comprising that drug, more particularly, to a drug for improving the resiliency and body of hair by activating hair-associated cells and hair follicle cells, and even more particularly, to increasing the expression of hair-associated genes, and to a hair cosmetic containing that drug.

### BACKGROUND ART

Concerns relating to hair involve the health of hair roots and hair follicles in the manner of thinning hair and hair loss, and the physical properties of hair in the manner hard hair, soft hair, hair lacking resiliency and body, split ends, frizzy hair or unmanageable hair. In addition, problems involving the physical properties of hair may be the result of damage caused by external factors resulting direct damage to hair such as hair permanent agents, bleaching agent treatment, exposure to ultraviolet light, exposure to air pollutants or combing friction, or may involve internal factors in the formation of hair shafts. With respect to hair care in the latter case, in addition to improving hair quality, it is also necessary to care for the hair by ensuring the favorable health of hair roots and hair follicles. Thus, even though the quality of hair may be considered to be poor, there are various methods of hair care according to the particular cause.

Hair itself is composed of a cuticle that covers the surface thereof, and internally a cortex, which accounts for the majority of the hair, and a central medulla. The cuticle has been clearly demonstrated to greatly contribute to the hardness, resiliency and body of hair (Sogabe, A. et al., J. Soc. Cosmet. Chem. Japan, Vol. 36, No. 3 (2002), pp. 207-216, "A Study of the Physical Properties of Hair, Part 1 - "Measurement of Hair Short Axis and Long Axis and Evaluation of Bending Stress"). The cuticle is known to be composed of various components including fibrous proteins such as acidic hair keratins Ha1 and Ha2 and basic keratins Hb1 and Hb2, modifying enzymes such as transglutaminase and peptidyl arginine deiminase, and a granular component in the form of tricohyaline S100. However, the relationship between these components and, for example, the resiliency and body of hair has yet to be fully understood. If those mechanisms that influence hair were adequately elucidated at the biochemical and molecular biological levels, it would be possible to provide hair improvers capable of demonstrating more prominent effects than existing hair improvers.

An example of hair cosmetics of the prior art that impart resiliency and body to hair is a hair treatment composition comprising a copolymer having a silyl group bonded by a reactive functional group as described in Japanese Unexamined Patent Publication (Kokai) No. 11-302129 that forms Si-O-Si siloxane bonds in the hair by hydrolysis resulting in the occurrence of crosslinking between copolymer molecules and thus imparting resiliency and body to the hair. However, these hair cosmetics of the prior art are coated onto the surface of hair, and do not improve the resiliency and body of hair by penetrating into hair or being absorbed into hair follicles and activating hair-associated cells or hair follicle cells.

On the other hand, a cell activator containing taurine as an active ingredient thereof, is described in Japanese Unexamined Patent Publication (Kokai) No. 2002-97116, while a cell activator and hair resiliency and body improver, containing N-methyltaurine as an active ingredient thereof, are described in International Publication No. WO 2002/034253. Although the actions and effects of these drugs are described as being control of hair cells, extension of the hair growth period, activation of hair cell proliferation and improvement of hair resiliency and body by activating cell proliferation, the effects of improving hair resiliency and body described here are still evaluated by testing the physical properties of hair (torsion torque). Thus, there is a need for a novel hair resiliency and body improver based on an evaluation of activity at the biochemical and molecular biological levels.

### DISCLOSURE OF THE INVENTION

An object of the present invention is to provide a novel hair resiliency and body improver based on an evaluation of activity at the biochemical and molecular biological levels.

As a result of investigating the correlation between hair resiliency and body (using hardness, and more specifically, Young's modulus, as an indicator thereof) and expression of hair-associated genes, the inventors of the present invention found that the greater the expression of hair keratin genes, and particularly hair keratin Ha2 gene, hair keratin Hb2 gene and/or keratin-associated proteins KAP5.1 to 5.5, and more preferably the greater the expression of hair keratin Ha2 gene, KAP5.1 gene, KAP5.2 gene or KAP5.4 gene, the higher the Young's modulus of the hair, namely the greater the feeling of resiliency and body of the hair, thereby leading to filing of a patent application entitled "Hair Evaluation Method Using Hair Keratin Genes as Indicators" (Japanese Patent Application No. 2004-164596).

As a result of newly developing an assay system for evaluating the effects of various drugs on the expression of hair-associated genes and using this assay system to extensively study the effects of various compounds on the expression of hair-associated genes, the inventors of the present invention found that specific 2-aminothiazole and 3-aminoisoxazole derivatives have the effect of increasing the expression of hair-associated genes, thereby leading to completion of the present invention.

Namely, in a first aspect thereof, the present invention relates to a hair resiliency and body improver comprising as the active ingredient one or more types of compounds selected from the group consisting of 2-aminothiazole derivatives represented by the following general formula (1) and pharmaceutically acceptable salts thereof: 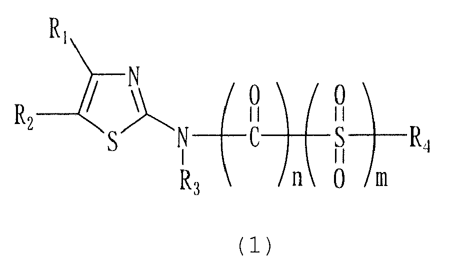 wherein, R₁, R₂, R₃ and R₄ independently represent a hydrogen atom, linear or branched alkyl group having 1 to 8 carbon atoms, linear or branched alkenyl group having 2 to 8 carbon atoms, cycloalkyl group having 3 to 8 carbon atoms, aralkyl group having 7 to 10 carbon atoms, aryl group having 6 to 14 carbon atoms or heteroaryl group having 5 to 14 members, and n and m represent 0 or 1, provided that when n is 1, m is 0 and R₃ is a hydrogen atom or methyl group and when m is 1, n is 0 and R₃ is a hydrogen atom or methyl group.

In addition, the present invention relates to a hair cosmetic comprising as the active ingredient one or more types of compounds selected from the group consisting of a 2-aminothiazole derivative represented by general formula (1) above, wherein R₂ is a hydrogen atom, and pharmaceutically acceptable salts thereof.

In addition, the present invention relates to a method for improving hair resiliency and body comprising the step of applying a hair cosmetic, containing one or more types of compounds selected from the group consisting of 2-aminothiazole derivatives and pharmaceutically acceptable salts thereof, to the hair or scalp.

In addition, the present invention relates to a method for preparing a hair cosmetic comprising the step of producing a hair cosmetic by adding one or more types of compounds selected from the group consisting of 2-aminothiazole derivatives and pharmaceutically acceptable salts thereof to an aqueous phase or oily phase.

In a second aspect thereof, the present invention relates to a hair cosmetic comprising as the active ingredient one or more types of compounds selected from the group consisting of 3-aminoisoxazole derivatives represented by the following general formula (2) and pharmaceutically acceptable salts thereof: 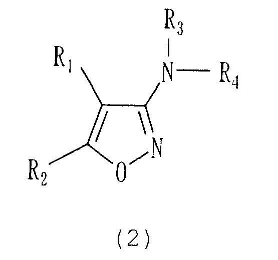 wherein, R₁ represents a hydrogen atom or methyl group, R₂ represents a hydrogen atom, linear or branched alkyl group having 1 to 8 carbon atoms, linear or branched alkenyl group having 2 to 8 carbon atoms, cycloalkyl group having 3 to 8 carbon atoms, aralkyl group having 7 to 10 carbon atoms or aryl group having 6 to 14 carbon atoms, R₃ represents a hydrogen atom or methyl group, and R₄ represents a hydrogen atom, linear or branched alkyl group having 1 to 8 carbon atoms, linear or branched alkenyl group having 2 to 8 carbon atoms, cycloalkyl group having 3 to 8 carbon atoms, aralkyl group having 7 to 10 carbon atoms, aryl group having 6 to 14 carbon atoms, alkylcarbonyl group having 2 to 8 carbon atoms, or arylcarbonyl group having 7 to 10 carbon atoms, and the aryl moiety of the aralkyl groups, aryl groups and arylcarbonyl groups may be independently substituted with an alkyl group having 1 to 3 carbon atoms, an alkoxy group having 1 to 3 carbon atoms or a hydroxyl group.

The hair cosmetic is preferably a hair resiliency and body improver.

In addition, the present invention relates to a method for improving hair resiliency and body comprising the step of applying a hair cosmetic, containing one or more types of compounds selected from the group consisting a 3-aminoisoxazole derivative represented by general formula (2) above and pharmaceutically acceptable salts thereof, to the hair or scalp.

In addition, the present invention relates to a method for preparation of a hair cosmetic comprising the step of producing a hair cosmetic by adding one or more types of compounds selected from the group consisting of 3-aminoisoxazole derivatives represented by general formula (2) above and pharmaceutically acceptable salts thereof to an aqueous phase or oily phase.

The active ingredient of the hair resiliency and body improver and hair cosmetic of the present invention in the form of a 2-aminothioazole derivative, 3-aminoisoxazole derivative or pharmaceutically acceptable salts thereof penetrates into the hair or is absorbed into hair follicles as a result of using on the hair or scalp, thereby increasing intracellular expression of the keratin-associated protein, KAP5, and making it possible to improve the resiliency and body of hair.

### BRIEF DESCRIPTION OF THE DRAWINGS

FIG. 1 is a schematic drawing showing the construction and configuration of a KAP5.1 reporter plasmid. FIG. 1-A is a drawing showing PCR amplification of a DNA fragment containing KAP5.1 gene and KAP5.2 gene, and FIG. 1-B is a drawing showing cloning of the amplified DNA fragment to a reporter plasmid vector pGL-3basic.
FIG. 2 is a construction drawing of a KAP5.1 reporter plasmid continuing from FIG. 1, and shows the deletion of a KAP5.1 gene translation region (starting with the starting codon) by subcloning.
FIG. 3 is a drawing showing regulation of expression of a KAP5 gene group.
FIG. 4 is a drawing showing a comparison of amino acid sequences of deduced encoded proteins of human KAP5.1 to KAP5.5 genes.

### BEST MODE FOR CARRYING OUT THE INVENTION

The present invention relates to a drug and hair cosmetic that improve hair resiliency and body by activating hair-associated cells and hair follicle cells, and more particularly, by increasing expression of hair-associated genes, and provides a novel hair resiliency and body improver and hair cosmetic based on evaluation of activity at the biochemical and molecular biological levels, and more particularly, based on an assay of effects regulating the expression of keratin-associated protein KAP5 genes.

In the previously filed Japanese Patent Application No. 2004-164596 entitled "Evaluation Method of Hair Property Using Hair Keratin Genes as Indicators", the inventors of the present invention measured Young's modulus as an indicator of hair hardness to investigate a correlation between the hardness of hair and various types of genes extracted from hair, and conducted a statistical analysis of the relationship between the results of those measurements and expressed amounts of various genes as measured by RT-PCR using Spearman's rank correlation coefficient (Zar, J.H., J. Amer. Stat. Assoc., 67: 578-580, 1970, "Significance testing of the Spearman rank correlation coefficient"). As a result, the Young's modulus of hair was found to be statistically significantly higher if the expressed amounts of hair keratin Ha2, KAP5.1, KAP5.2 and KAP5.4 genes are high. In addition, the Young's modulus of hair was also found to be higher, although not statistically significantly higher, even in the case of large expressed amounts of hair keratin Hb2 gene. On the other hand, there was no correlation with hair hardness for other constituent proteins of the cortex such as hair keratin Ha1. Thus, in comparison with the cortex, a higher proportion of constituent proteins of the cuticle and associated genes thereof were suggested to be involved in hair hardness.

Hair keratin Ha2 gene (NCBI GenBANK Source "X90761") encodes acidic protein that is a constituent member of the keratin gene family. Hair keratin Ha2 is a type I keratin, and composes hair, nails and the like by forming a heterodimer with type II keratin. Type I keratin is localized on the q12-q21 region of chromosome 17.

Hair keratin Hb2 gene (NCBI GenBANK Source "Y19207") encodes a basic protein that is a constituent member of the keratin gene family. Hair keratin Hb2 is a type II keratin, and composes hair, nails and the like by forming a heterodimer with type I keratin. Type II keratin is localized on the q13 region of chromosome 12.

KAP5.1 to KAP5.5 genes (SEQ ID NO. 1 to 5), belonging to the human keratin-associated protein (KAP) 5 family (Homo sapiens genomic DNA, chromosome II clone: RP11-684B2, complete sequences; Accession No.: AP000867), are human ESTs for which only the nucleotide sequences are known, and there have been no reports regarding their functions. The relationship between increased expression of KAP5.1 to KAP5.5 genes and hair hardness was unknown. Although not constrained to a particular theory, since, for example, deduced proteins encoded by KAP5.2 gene are cysteine-rich (about 35.5%), and are composed of 186 amino acids rich in the low molecular weight amino acids of glycine and serine (18.8% and 24.2%, respectively), they do not form an α-helix or other secondary structure and easily enter between keratin fibers, and since it is also plausible that they form a large number of hydrogen bonds by means of the OH groups of the large number of serine residues, they have the potential to greatly contribute to hair mechanical strength. Other KAP5 genes are similarly rich in cysteine, glycine and serine, their expression products have a structure similar to that of KAP5.2, and are also considered to greatly contribute to hair mechanical strength. The following Table 1 shows the partial amino acid compositions of deduced proteins encoded by KAP5.1 to KAP5.5 genes.

**Table 1 Characteristics of Human KAP5.x Genes**

| | Amino acid residues | MW (kDa) | pI | Contents | | | |
|---|---|---|---|---|---|---|---|
| | | | | Cys | Ser | Gly | Pro |
| KAP5.1 | 164 | 15.0 | 8.06 | 59 (36.0%) | 35 (21.3%) | 37 (12.6%) | 8 (4.9%) |
| KAP5.2 | 186 | 17.4 | 8.30 | 66 (35.5%) | 45 (24.2%) | 35 (18.8%) | 10 (5.4%) |
| KAP5.3 | 168 | 16.1 | 8.34 | 60 (35.7%) | 39 (23.2%) | 27 (16.1%) | 10 (6.0%) |
| KAP5.4 | 201 | 17.9 | 8.20 | 66 (32.8%) | 37 (18.4%) | 59 (29.4%) | 8 (4.0%) |
| KAP5.5 | 155 | 14.5 | 8.17 | 55 (35.5%) | 35 (22.6%) | 30 (19.4%) | 8 (5.5%) |

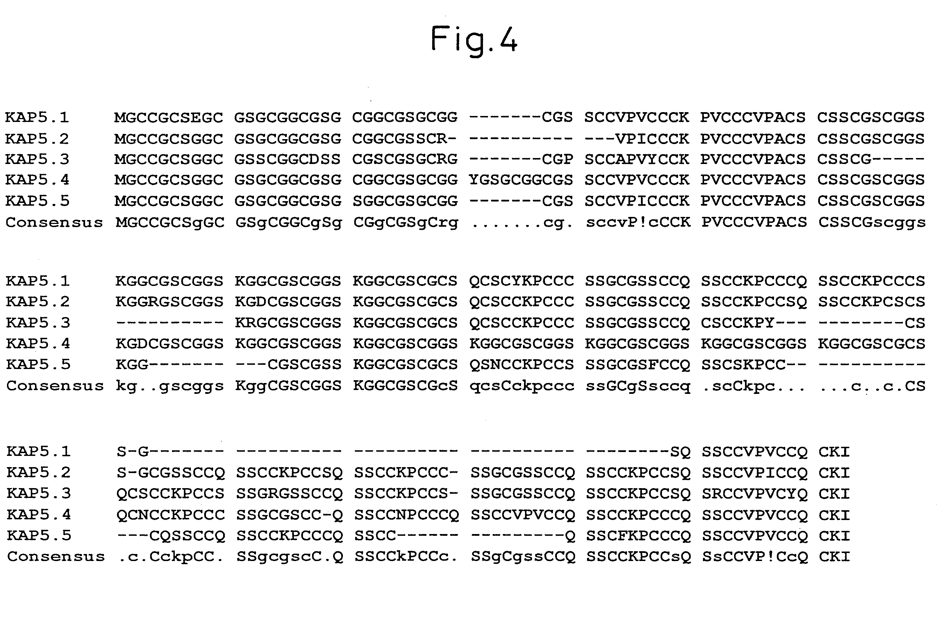

KAP5.1 to KAP5.5 genes (SEQ ID NO. 1 to 5) belong to the KAP5 family of human keratin-associated proteins. This gene group has a comparatively high level of mutual homology. The high degrees of homology between amino acid sequences of deduced proteins encoded by each gene are clear from FIG. 4 and Table 2 below.

**Table 2 Homology of Amino Acid Sequences of Human KAP5.x Genes**

| | KAP5.1 | KAP5.2 | KAP5.3 | KAP5.4 |
|---|---|---|---|---|
| KAP5.2 | 86 | | | |
| KAP5.3 | 72 | 74 | | |
| KAP5.4 | 69 | 68 | 52 | |
| KAP5.5 | 81 | 70 | 72 | 63 |

The inventors of the present invention newly constructed a reporter assay system for evaluating the effects of various drugs on the expression of the hair-associated gene KAP5.1 gene, among the hair keratin Ha2 and Hb2 genes and hair keratin-associated protein KAP5.1, KAP5.2 and KAP.4 genes, which have been observed to demonstrate a correlation with the hardness (Young's modulus) of hair. Extensive studies were then conducted on the effects of various compounds on the expression of KAP5.1 gene using this assay system. As a result, 2-aminothiazole derivatives and pharmaceutically acceptable salts thereof were found to have the effect of increasing expression of KAP5.1 gene.

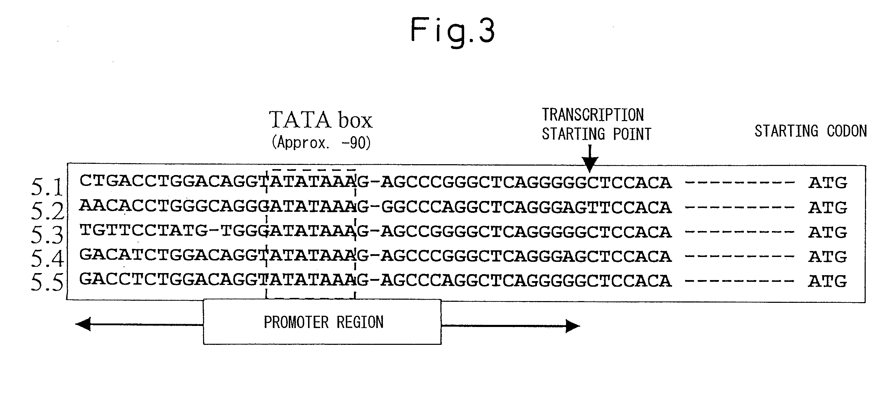

As was previously described, KAP5.1 to KAP5.5 genes (SEQ ID NO. 1 to 5) constitute a group of genes belonging to the human keratin-associated protein KAP5 family that have comparatively high levels of mutual homology. In addition, as can be understood from FIG. 3, the sequence of the promoter region of KAP5.1 gene is extremely similar to the sequences of the promoter regions of other KAP5.2 to KAP5.5 genes, and there is a high likelihood that their control mechanisms are the same. Thus, drugs that increase expression of KAP5.1 gene are considered to have an extremely high possibility of also increasing expression of other KAP5 genes.

Moreover, as was also previously described, since the larger the expressed amount of KAP5.1 gene, the greater the statistically significant increase in Young's modulus, drugs that increase expression of KAP5.1 gene improve hair hardness, thereby improving hair resiliency and body.

The 2-aminothiazole derivatives of the present invention are known compounds that can be easily acquired. In addition, they can be easily synthesized using known methods from easily acquired 2-aminothiazole and the like.

Although the 2-aminothiazole derivatives of the present invention are known to be applied as nitrification inhibitors (Japanese Examined Patent Publication (Kokoku) No. 46-9208), antimicrobials or bactericidals (International Publication No. WO 96/16650), pruritis therapeutic drugs (Japanese Unexamined Patent Publication (Kokai) No. 2002-241308), and cyclin-dependent protein kinase cdk5 and cdk2 as well as glycogen synthase kinase 3 inhibitors (Japanese Unexamined Patent Publication (Kokai) No. 2002-338556), their effect of increasing the expression of hair-associated genes and their improvement of hair resiliency and body have been completely unknown. In addition, although pharmaceutical compositions containing thiazole derivatives have been disclosed for the treatment of hair loss, thinning hair and baldness (Japanese Unexamined Patent Publication (Kokai) No. 2002-338556), there has been no mention of increased expression of hair-associated genes, and the fact that substituent R₁ of these aminothiazole derivatives (corresponding to substituent R₂ in the present invention) does not include a hydrogen atom, they differ from the 2-aminothiazole derivatives of the present invention.

Examples of specific substance names of the 2-aminothiazole derivatives of the present invention include 2-aminothiazole, 2-amino-4-methylthiazole, 2-amino-5-methylthiazole, 2-amino-4,5-dimethylthiazole, 2-acetamidothiazole, 2-acetamido-4-methylthiazole, 2-acetamido-5-methylthiazole, 2-acetamido-4,5-dimethylthiazole, 2-acetamido-4,5-diphenylthiazole, 2-propionylaminothiazole, 2-butyrylaminothiazole, 2-valerylaminothiazole, 2-propionylamino-4-methylthiazole, 2-butyrylamino-4-methylthiazole, 2-valerylamino-4-methylthiazole, 2-methylaminothiazole, 2-dimethylaminothiazole, 2-methylsulfonylamino-4,5-diphenylthiazole and 2-benzenesulfonylamino-4,5-diphenylthiazole and the like. Among these, 2-aminothiazole, 2-acetamidothiazole and 2-acetamido-4-methylthiazole are preferable.

Although the 3-aminoisoxazole derivatives of the present invention are known to be applied as herbicides (Japanese Examined Patent Publication (Kokoku) No. 54-44723), their effect of increasing expression of hair-associated genes and their improvement of hair resiliency and body has been completely unknown.

Examples of specific substance names of the 3-aminoisoxazole derivatives of the present invention include 3-aminoisoxazole, 3-amino-4-methylisoxazole, 3-amino-5-methylisoxazole, 3-amino-5-ethylisoxazole, 3-amino-5-n-propylisoxazole, 3-amino-5-cyclopropylisoxazole, 3-amino-5-n-butylisoxazole, 3-amino-5-tert-butylisoxazole, 3-amino-5-n-pentylisoxazole, 3-amino-5-isoamylisoxazole, 3-amino-5-n-hexylisoxazole, 3-amino-5-cyclohexylisoxazole, 3-amino-5-n-octylisoxazole, 3-amino-5-phenylisoxazole, 3-amino-5-(p-methoxyphenyl)isoxazole, 3-amino-5-(p-methoxyphenethyl)isoxazole, 3-amino-4,5-dimethylisoxazole, 3-acetamidoisoxazole, 3-acetamido-4-methylisoxazole, 3-acetamido-5-methylisoxazole, 3-acetamido-5-tert-butylisoxazole, 3-(N-acetyl-N-methylamino)-5-methylisoxazole, 3-(N-acetyl-N-methylamino)-5-tert-butylisoxazole, 3-acetamido-4,5-dimethylisoxazole, 3-propionylaminoisoxazole, 3-propionylamino-5-methylisoxazole, 3-propionylamino-5-tert-butylisoxazole, 3-(N-propionyl-N-methylamino)-5-methylisoxazole, 3-(N-propionyl-N-methylamino)-5-tert-butylisoxazole, 3-butyrylamino-5-methylisoxazole, 3-butyrylamino-5-tert-butylisoxazole, 3-(N-methylamino)-5-methylisoxazole, 3-(N,N-dimethylamino)-5-tert-butylisoxazole and the like.

A "pharmaceutically acceptable salt" refers to a salt that retains nearly equal biological activity as the drug compound and has low toxicity. Examples of such salts include, but are not limited to, addition salts formed with inorganic acids such as hydrochlorides, hydrobromides, sulfates, phosphates and nitrates, and addition salts formed with organic acids such as acetates, oxalates, tartrates, succinates, malates, fumarates, maleates, ascorbates and benzoates.

There are no particular limitations on the form of the hair resiliency and body improver and hair cosmetic containing a 2-aminothiazole derivative or 3-aminoisoxazole derivative and pharmaceutically acceptable salts thereof of the present invention, and the form may be any form ordinarily used for improving hair resiliency and body, examples of which include a liquid, emulsion, emulsified cream, cream, gel, wax, mist, foam and aerosol. The hair resiliency and body improver and hair cosmetic of the present invention can be applied to, for example, products such as hair liquids, hair tonics, hair creams, mousses, treatments, hair restoration agents, shampoos, rinses, creams, emulsions and packs.

Although varying according to the hair cosmetic, the incorporated amount of the 2-aminothiazole derivative or 3-aminoisoxazole derivative and pharmaceutically acceptable salts thereof of the present invention is typically 0.001 to 20% by weight and preferably 0.01 to 5% by weight based on the total amount of the hair cosmetic.

The hair resiliency and body improver and hair cosmetic of the present invention can be prepared by incorporating conventionally formulated components of cosmetics, over-the-counter medicines and pharmaceuticals in accordance with ordinary methods as necessary within a range that does impair the effects of the present invention. Examples of conventionally formulated components include powdered ingredients, liquid oils, solid oils, waxes, hydrocarbon oils, higher fatty acids, higher alcohols, ester oils, silicone oils, anionic surfactants, cationic surfactants, amphoteric surfactants, nonionic surfactants, moisturizers, watersoluble polymers, thickeners, coating agents, ultraviolet absorbers, metal ion chelating agents, lower alcohols, polyvalent alcohols, sugars, amino acids, organic amines, polymer emulsions, pH adjusters, skin nutrients, vitamins, antioxidants, antioxidation assistants, fragrances and water.

Specific examples of ingredients able to be incorporated are indicated below. The hair resiliency and body improver and hair cosmetic is produced in accordance with ordinary methods corresponding to the desired form by incorporating one type or two or more types of the ingredients indicated below.

Specific examples of ingredients able to be incorporated in the hair resiliency and body improver and hair cosmetic include powders such as polyethylene powder, methyl polymethacrylate powder or Nylon powder; inorganic pigments such as titanium dioxide, zinc oxide, synthetic mica, talc, kaolin, zeolite or boron nitride; oils such as camellia oil, macadamia nut oil, olive oil or castor oil; waxes such as jojoba oil, beeswax, candelilla wax, carnauba wax or lanolin; hydrocarbon oils such as liquid paraffin, squalane, paraffin, ceresin, vaseline or microcrystalline wax; higher fatty acids such as lauric acid, myristic acid, palmitic acid, stearic acid or isostearic acid; higher alcohols such as cetyl alcohol, stearyl alcohol, isostearyl alcohol or octyl decanol; ester oils such as isopropyl myristate or diisostearyl malate; silicone oils such as methyl polysiloxane; anionic surfactants such as higher fatty acid soaps, higher alkyl sulfate ester salts, N-acylsuccinate or POE-alkyl ether sulfates; cationic surfactants such as alkyl trimethyl ammonium chloride, dialkyl dimethyl ammonium chloride or benzalkonium chloride; amphoteric surfactants such as betaine alkyl dimethylamino acetate or betaine alkyl amidopropyl dimethylamino acetate; nonionic surfactants such as polyoxyethylene types, polyvalent alcohol ester types or ethylene oxide-propylene oxide block copolymers; moisturizers such as polyethylene glycol, propylene glycol, glycerin, 1,3-butylene glycol, sorbitol, sodium hyaluronate or sodium pyrrolidone carboxylate; watersoluble polymers such as quince seed gum, xanthane gum, carboxymethyl cellulose or carboxyvinyl polymer; coating agents such as polyvinyl alcohol, polyvinyl pyrrolidone, nitrocellulose or polymer silicone; ultraviolet absorbers such as PABA-based, cinnamic acid-based, salicylic acid-based, benzophenone-based, benzotriazole-based or dibenzoylmethane-based ultraviolet absorbers; metal ion chelating agents such as sodium salts of ethylenediamine tetraacetic acid (EDTA); photostabilizers such as 4,5-dimorpholinopyridazinone; lower alcohols such as ethanol, propanol, isopropanol, isobutyl alcohol or b-butyl alcohol; polyvalent alcohols such as 1,3-butylene glycol, xylitol, sorbitol, diethylene glycol or polyglycerin; monosaccharides such as triose, tetrose, pentose, hexose, deoxyose, aminosaccharides or uronic acid; polysaccharides such as cellulose or chondroitin sulfate; amino acids and derivatives thereof such as glycine, serine, lysine, cystine, cysteine, methionine, glutamic acid or proline; organic amines such as monoethanolamine, diethanolamine or triethanolamine; pH adjusters such as lactic acid-sodium lactate or citric acid-sodium citrate; vitamins and derivatives thereof such as vitamin A, vitamin B₁, vitamin B₂, vitamin C, vitamin E, biotin or nicotinic amide; antioxidants such as dibutyl hydroxytoluene, butyl hydroxyanisole, gallic acid ester, hypotaurine or thiotaurine; antiseptics such as paraoxybenzoic acid esters or phenoxyethanol; propellants such as various types of liquefied petroleum gas (LPG), nitrogen gas or carbonic acid gas; antiphlogistics such as glycyrrhizic acid, glycyrrhetinic acid, hinokithiol or zinc oxide; protease inhibitors such as tranexamic acid; whiteners such as albutin, ascorbic acid glucoside, 4-methoxysalicylates, ellagic acid or rucinol; anti-wrinkle agents such as retinol or α-hydroxyacids; activators such as royal jelly, photosensitizers or cholesterol derivatives; circulation promoters such as Swertia japonica extract, γ-oryzanol, cepharanthine, vitamin E and derivatives thereof, nicotinic acid benzyl ester, adenosine or minoxidil; local stimulants such as capsaicin, cayenne pepper tincture, ginger tincture, cantharides tincture or nonylic acid vanillylamide; anti-androgenic agonists such as estradiol or ethynyl estradiol; pantothenic acid and derivatives thereof; hair follicle activators such as allantoin or photosensitizer 301; keratin exfoliation and dissolving agents such as salicylic acid, sulfur, resorcin or selenium sulfide; anti-seborrhea agents such as vitamin B₆ and derivatives thereof; antipruritics such as diphenhydramine hydrochloride, chlorpheniramine maleate, camphor or menthol; and various types of extracts such as those of phellodendron bark, coptis rhizome, *Lithospermum erythrorhizon,* Chinese peony, birch, sage, loquat, carrot, aloe, tree mallow, iris, grape, coix seed, luffa, lily, saffron, Cnidium rhizome, ginger, bush clover, restharrow root, garlic, cayenne pepper, orange peel, angelica or algae.

The following provides a more detailed explanation of the present invention through specific examples thereof.

### EXAMPLES

### Example 1 - Construction of KAP5.1 Reporter Plasmid

The transcriptional regulatory region of KAP5.1, which has been observed to demonstrate a correlation with hair hardness (Young's modulus), was amplified by PCR followed by the construction of a reporter plasmid.

### A. PCR Amplification of DNA Fragment

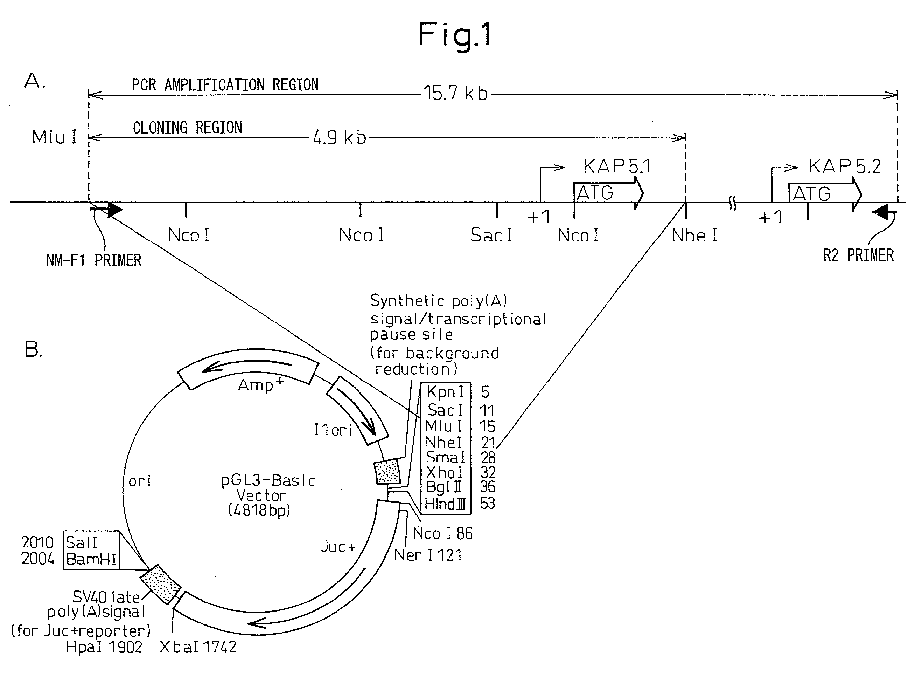

A DNA fragment of about 15 kb containing KAP5.1 gene and KAP5.2 gene was amplified by PCR using an NM-F1 primer (SEQ ID NO. 6), in which the recognition sites of restriction endonuclease NotI and MluI were added to the 5'-terminal thereof, and an R2 primer (SEQ ID NO. 7), and using commercially available human genomic DNA (obtained from Promega) as the template (FIG. 1-A).

Furthermore, the conditions of the PCR reaction were as indicated below.

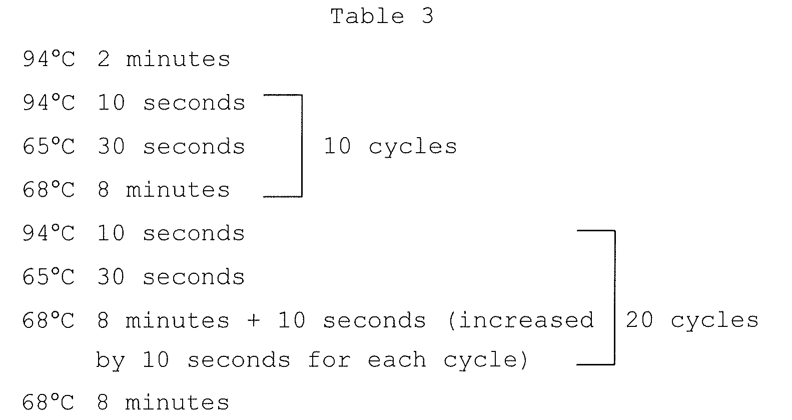

The NM-F1 primer (SEQ ID NO. 6) and the R2 primer (SEQ ID NO. 7) were as shown in the table below. Furthermore, the numbers shown in the table indicate the locations of the sequence numbers of Homo sapiens genomic DNA, chromosome 11 clone: RP11-684B2, complete sequences, Accession No.: AP000867.5.

**Table 4**

| SEQUENCE 6: | | |
|---|---|---|
| TTTGCGGCCGC | ACGCGT | **TTTCAGTAATGTGCCCCTCTTATGCT** |
| NotI site | MluI site | **Specific sequence in 5'-terminal upstream region of KAP5.1 gene** |
| 51039 **TTTCAGTAATGTGCCCCTCTTATGCT** 51064 | | |

| SEQUENCE 7: | | |
|---|---|---|
| 66762 **AGGTTGAAGTGATGAGGTCAGGAAAG** 66737 | | |
| **Specific sequence in 3'-terminal downstream region of KAP5.2 gene** | | |

### B. Cloning of DNA Fragment to Vector

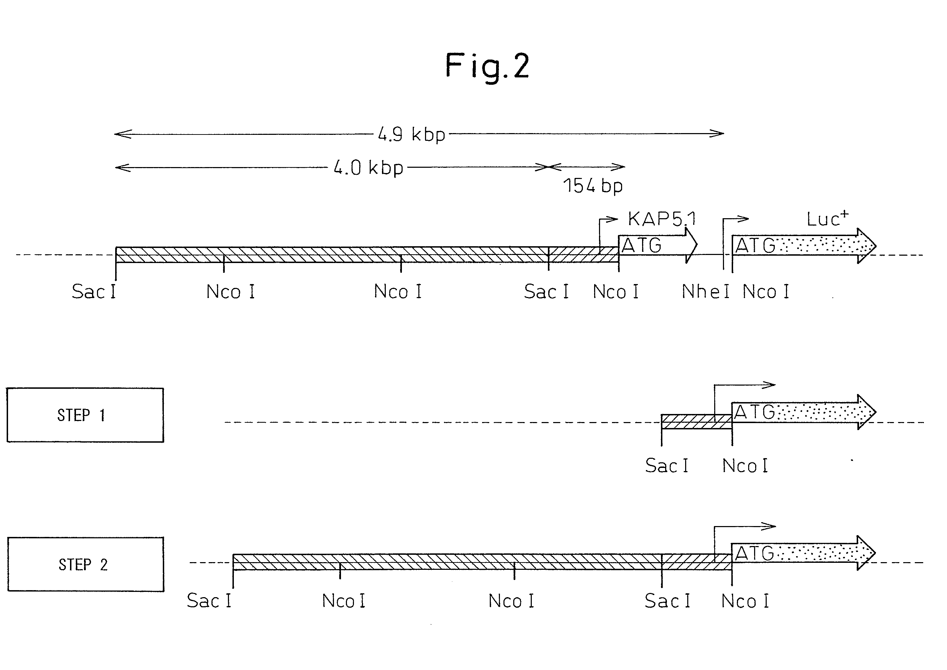

The amplified DNA fragment was double-digested with restriction endonucleases MluI and NheI, and a DNA fragment of about 4.9 kb was recovered following separation by agarose gel electrophoresis. This DNA fragment was cloned between the MluI and NheI sites of reporter vector pGL-3basic (obtained from Promega) having a gene that encodes firefly luciferase (FIG. 1-B). Reporter plasmid pGL-3-KAP5.1 containing the 5' upstream region (SEQ ID NO. 8) of KAP5.1 was then constructed by subcloning [Step 1: subcloning of SacI/NcoI fragment (154 bp, FIG. 2) to pGL-3basic; Step 2: insertion of ScaI fragment (about 4.0 kbp, FIG. 2) into resulting plasmid] (FIG. 2).

### Example 2 - KAP5.1 Reporter Assay

8 × 10⁵ immortalized outer root sheath (IORS) cells (obtained in accordance with Japanese Unexamined Patent Publication (Kokai) No. 2000-89) were seeded into a 75 cm² flask and cultured for 6 days under conditions of 37°C and 5% CO₂. Keratinocyte-SFM medium (Gibco) was used as the medium.

Day 1: Plasmid DNA Transfection (transient). The cultured IORS cells were dually transformed with the previously constructed reporter plasmid pGL3-KAP5.1 (firefly luciferase) and commercially available internal standard plasmid pRL-TK (renilla luciferase) in accordance with the manual provided with Effectene Transfection Reagent (Qiagen).

Day 2: The transformed cells were re-seeded into in a 24-well multiwell plate at 10 to 16 × 10⁴ cells per well and cultured overnight under conditions of 37°C and 5% CO₂.

Day 3: The medium in each well was replaced with a basal medium in which each drug was dissolved followed by culturing overnight under conditions of 37°C and 5% CO₂.

Day 4: Assay. Luciferase activity was assayed for each well using the Dual-Luciferase Reporter Assay System (Promega) in accordance with the manual provided by the manufacturer. The AutoLumat LB953 (Berthold) was used for the luminometer.

An assay was carried out under the same conditions with the exception of not containing drug as a negative control [K-SFM(-)]. The assay results are shown in Table 5. The effect of increasing gene expression are represented in the form of the rate of increase (ratio based on a value of 100 for the negative control).

**Table 5**

| Drug Name | Rate of Increase |
|---|---|
| 100 ppm 2-acetamidothiazole | 180 |
| 10 ppm 2-acetamidothiazole | 130 |
| 100 ppm 2-acetamido-4-methylthioazole | 150 |
| 10 ppm 2-acetamido-4-methylthiazole | 110 |
| 100 ppm 2-aminothiazole | 140 |
| 10 ppm 2-aminothiazole | 110 |
| 300 ppm 3-amino-5-methylisoxazole | 160 |
| 100 ppm 3-amino-5-methylisoxazole | 140 |
| 10 ppm 3-amino-5-methylisoxazole | 120 |
| 300 ppm 3-acetamido-5-methylisoxazole | 125 |
| 100 ppm 3-acetamido-5-methylisoxazole | 110 |
| 10 ppm 3-acetamido-5-methylisoxazole | 100 |
| Negative control [K-SFM(-)] | 100 |

As shown in Table 5, the 2-aminothiazole derivatives or 3-aminoisoxazole derivatives of the present invention were observed to demonstrate effects that increase expression of KAP5.1 gene.

Although assay results were shown for only KAP5.1 in this example, as was previously stated, since the sequence of the promoter region of KAP5.1 gene and the sequences of the promoter regions of other KAP5.2 to KAP5.5 genes are extremely similar (FIG. 3) and their control mechanisms have a high possibility of being the same, drugs that increase expression of KAP5.1 are considered to also increase expression of other KAP5 genes, and therefore improve hair resiliency and body.

### Example 3 - Preparation of Hair Cosmetics

The following indicates formulation examples of the hair cosmetic of the present invention. Furthermore, all incorporated amounts are indicated in percent by weight. Wax hardness was measured with the MAX ME-415 Curd Meter manufactured by I-Techno Engineering Co., Ltd. (diameter: 5.6 mm; speed: 7 sec/inch; load: 200 g; temperature: 37°C). Viscosity was measured at a temperature of 30°C with the 1287 Single Cylinder Rotary Viscometer manufactured by Shibaura Systems Co., Ltd. Furthermore, the model VS-H1 (rotor no. 6/10 rpm) was used for high viscosity preparations such as gels and treatments, while the model VS-A1 (rotor no. 1/60 rpm) was used for low viscosity preparations such as mists, hair liquids, mousses, hair tonics and hair restoration agents.

### Example 3.1 - Wax

A wax was prepared in accordance with ordinary methods containing a drug of the present invention according to the formula indicated below. The resulting wax was an emulsified wax and had a hardness of 20. This wax had a favorable feel during use and was observed to improve hair resiliency and body.

| | |
|---|---|
| 2-acetamidothiazole | 0.1 |
| Liquid paraffin | 10 |
| Microcrystalline wax | 10 |
| Dimethyl polysiloxane | 4 |
| Stearyl alcohol | 4 |
| Propylene glycol | 10 |
| Carnauba wax | 3 |
| Isostearic acid | 0.5 |
| Stearic acid | 4.5 |
| Pentaerythritol tetra(2-ethylhexanoate) | 2 |
| Polyoxyethylene-hydrogenated castor oil | 3 |
| Polyoxyethylene oleyl ether phosphate | 2 |
| Self-emulsified glycerin monostearate | 3 |
| Triethanolamine | 1 |
| Paraoxybenzoic acid ester | As suitable |
| Sodium polyacrylate | As suitable |
| Purified water | Balance |
| Fragrance | As suitable |

### Example 3.2 - Mist

A mist was prepared in accordance with ordinary methods containing a drug of the present invention according to the formula indicated below. The resulting mist was a mist (aerosol) and had a viscosity of 5. This mist had a favorable feel during use and was observed to improve hair resiliency and body.

| | |
|---|---|
| 2-acetamidothiazole | 1 |
| Acrylic resin alkanolamine solution | 12 |
| Lauric acid diethanolamide | 0.5 |
| Ethanol | 57 |
| Polyoxyethylene oleyl ether | 0.5 |
| Purified water | Balance |

### Example 3.3 - Gel

A gel was prepared in accordance with ordinary methods containing a drug of the present invention according to the formula indicated below. The resulting gel was a transparent solid (gel) and had a viscosity of 30000. This gel had a favorable feel during use and was observed to improve hair resiliency and body.

| | |
|---|---|
| 2-acetamidothiazole | 1 |
| Behenyl alcohol | 1 |
| Glycerin | 5 |
| 1,3-butylene glycol | 5 |
| Polyoxyethylene-hydrogenated castor oil | 40 |
| succinate (50 E.O.) | |
| Potassium hydroxide | 1 |
| 2-ethylhexyl paramethoxycinnamate | 0.1 |
| Purified water | Balance |
| Fragrance | As suitable |

### Example 3.4 - Hair Liquid

A hair liquid was prepared in accordance with ordinary methods containing a drug of the present invention according to the formula indicated below. The resulting hair liquid was a transparent solid and had a viscosity of 8. This hair liquid had a favorable feel during use and was observed to improve hair resiliency and body.

| | |
|---|---|
| 2-acetamidothiazole | 3 |
| Ethanol | 55 |
| Propylene glycol | 5 |
| Polyoxyethylene-polyoxypropylene- | 25 |
| pentaerythritol ether (5 E.O.) | |
| 2-ethylhexyl paramethoxycinnamate | 0.5 |
| Pigment | As suitable |
| Purified water | Balance |
| Fragrance | As suitable |

### Example 3.5 - Treatment

A treatment was prepared in accordance with ordinary methods containing a drug of the present invention according to the formula indicated below. The resulting treatment was an emulsified cream and had a viscosity of 25000. This treatment had a favorable feel during use and was observed to improve hair resiliency and body.

| | |
|---|---|
| 2-acetamidothiazole | 0.5 |
| Liquid paraffin | 10 |
| Vaseline | 3 |
| Dimethyl polysiloxane | 2 |
| Propylene glycol | 10 |
| Polyoxypropylene (40) butyl ether | 2 |
| Pentaerythritol tetra(2-ethylhexanoate) | 3 |
| Polyoxyethylene-hydrogenated castor oil | 2 |
| Potassium hydroxide | 0.1 |
| Carboxyvinyl polymer | 0.3 |
| Purified water | Balance |
| Fragrance | As suitable |

### Example 3.6 - Mousse

A mousse was prepared in accordance with ordinary methods containing a drug of the present invention according to the formula indicated below. The ratio of undiluted liquid to propellant was 90/10. The resulting mousse was a foam (aerosol) and had a viscosity of 5. This mousse had a favorable feel during use and was observed to improve hair resiliency and body.

| | |
|---|---|
| Stock: 2-acetamidothiazole | 0.5 |
| Volatile isoparaffin | 0.5 |
| Ethanol | 10 |
| Polyoxyethylene-hydrogenated castor oil | 0.5 |
| Polyoxyethylene-polyoxypropylene decyl ether | 0.5 |
| Loquat leaf extract | 0.1 |
| Yuka Foamer SM | 10 |
| Purified water | Balance |
| Propellant: | |
| LPG | |

### Example 3.7 - Hair Tonic

A hair tonic was prepared in accordance with ordinary methods containing a drug of the present invention according to the formula indicated below. The resulting hair tonic was a transparent liquid and had a viscosity of 7. This hair tonic had a favorable feel during use and was observed to improve hair resiliency and body.

| | |
|---|---|
| 2-acetamidothiazole | 3 |
| Ethanol | 60 |
| Dipropylene glycol | 2 |
| Polyoxyethylene-hydrogenated castor oil | 0.5 |
| Lactic acid | As suitable |
| Sodium lactate solution | As suitable |
| Monoammonium glycyrrhizate | 0.1 |
| Nicotinic amide | 0.1 |
| DL-α-tocopherol acetate | 0.1 |
| L-menthol | 0.2 |
| Pigment | As suitable |
| Purified water | Balance |
| Fragrance | As suitable |

### Example 3.8 - Hair Tonic

A hair tonic was prepared in accordance with ordinary methods containing a drug of the present invention according to the formula indicated below. The resulting hair tonic was a transparent liquid and had a viscosity of 7. This hair tonic had a favorable feel during use and was observed to improve hair resiliency and body.

| | |
|---|---|
| 2-acetamido-4-methylthiazole | 2 |
| Ethanol | 60 |
| Dipropylene glycol | 2 |
| Polyoxyethylene-hydrogenated castor oil | 0.5 |
| Lactic acid | As suitable |
| Sodium lactate solution | As suitable |
| Monoammonium glycyrrhizate | 0.1 |
| Nicotinic amide | 0.1 |
| DL-α-tocopherol acetate | 0.1 |
| L-menthol | 0.2 |
| Pigment | As suitable |
| Purified water | Balance |
| Fragrance | As suitable |

### Example 3.9 - Hair Restoration Agent

A hair restoration agent was prepared in accordance with ordinary methods containing a drug of the present invention according to the formula indicated below. The resulting hair restoration agent was a transparent liquid and had a viscosity of 7. This hair restoration agent had a favorable feel during use and was observed to improve hair resiliency and body.

| | |
|---|---|
| 2-acetamidothiazole | 2 |
| Ethanol | 80 |
| Isostearyl alcohol | 2 |
| 1,3-butylene glycol | 3 |
| Polyoxyethylene-hydrogenated castor oil | 1 |
| Sodium lauryl sulfate | 0.3 |
| DL-malic acid | As suitable |
| β-glycyrrhetinic acid | 0.2 |
| Pantotenyl ethyl ether | 0.1 |
| Benzyl nicotinate | 0.1 |
| Nicotinic amide | 0.1 |
| DL-α-tocopherol acetate | 0.5 |
| Decyltetradecyl dimethylamine oxide solution (20%) | 5 |
| L-menthol | 1 |

### Example 3.10 - Hair Restoration Agent

A hair restoration agent was prepared in accordance with ordinary methods containing a drug of the present invention according to the formula indicated below. The resulting hair restoration agent was a transparent liquid and had a viscosity of 7. This hair restoration agent had a favorable feel during use and was observed to improve hair resiliency and body.

| | |
|---|---|
| 2-acetamido-4-methylthiazole | 2 |
| Ethanol | 80 |
| Isostearyl alcohol | 2 |
| 1,3-butylene glycol | 3 |
| Polyoxyethylene-hydrogenated castor oil | 1 |
| Sodium lauryl sulfate | 0.3 |
| DL-malic acid | As suitable |
| β-glycyrrhetinic acid | 0.2 |
| Pantotenyl ethyl ether | 0.1 |
| Benzyl nicotinate | 0.1 |
| Nicotinic amide | 0.1 |
| DL-α-tocopherol acetate | 0.5 |
| Decyltetradecyl dimethylamine oxide solution (20%) | 5 |
| L-menthol | 1 |

### Example 3.11 - Hair Lotion

A hair lotion was prepared in accordance with ordinary methods containing a drug of the present invention according to the formula indicated below. The resulting hair lotion was a transparent liquid and had a viscosity of 3000. This hair lotion had a favorable feel during use and was observed to improve hair resiliency and body.

| | |
|---|---|
| 2-acetamido-4-methylthiazole | 0.1 |
| Ethanol | 50 |
| Dipropylene glycol | 5 |
| Polyoxyethylene-hydrogenated castor oil | 0.5 |
| Malic acid | As suitable |
| Monoammonium glycyrrhizate | 0.1 |
| Nicotinic amide | 0.1 |
| DL-α-tocopherol acetate | 0.1 |
| L-menthol | 0.2 |
| Crosslinked N,N-dimethylacrylamide/sodium 2-acrylamido-2-methylpropane sulfonate | 0.3 |
| copolymer | |
| Pigment | As suitable |
| Purified water | Balance |
| Fragrance | As suitable |

### Example 3.12 - Wax

A wax was prepared in accordance with ordinary methods containing a drug of the present invention according to the formula indicated below. The resulting wax was an emulsified wax and had a hardness of 20. This wax had a favorable feel during use and was observed to improve hair resiliency and body.

| | |
|---|---|
| 3-amino-5-methylisoxazole | 0.1 |
| Liquid paraffin | 10 |
| Microcrystalline wax | 10 |
| Dimethyl polysiloxane | 4 |
| Stearyl alcohol | 4 |
| Propylene glycol | 10 |
| Carnauba wax | 3 |
| Isostearic acid | 0.5 |
| Stearic acid | 4.5 |
| Pentaerythritol tetra(2-ethylhexanoate) | 2 |
| Polyoxyethylene-hydrogenated castor oil | 3 |
| Polyoxyethylene oleyl ether phosphate | 2 |
| Self-emulsified glycerin monostearate | 3 |
| Triethanolamine | 1 |
| Paraoxybenzoic acid ester | As suitable |
| Sodium polyacrylate | As suitable |
| Purified water | Balance |
| Fragrance | As suitable |

### Example 3.13 - Mist

A mist was prepared in accordance with ordinary methods containing a drug of the present invention according to the formula indicated below. The resulting mist was a mist (aerosol) and had a viscosity of 5. This mist had a favorable feel during use and was observed to improve hair resiliency and body.

| | |
|---|---|
| 3-amino-5-methylisoxazole | 1 |
| Acrylic resin alkanolamine solution | 12 |
| Lauric acid diethanolamide | 0.5 |
| Ethanol | 57 |
| Polyoxyethylene oleyl ether | 0.5 |
| Purified water | Balance |

### Example 3.14 - Gel

A gel was prepared in accordance with ordinary methods containing a drug of the present invention according to the formula indicated below. The resulting gel was a transparent solid (gel) and had a viscosity of 30000. This gel had a favorable feel during use and was observed to improve hair resiliency and body.

| | |
|---|---|
| 3-amino-5-methylisoxazole | 1 |
| Behenyl alcohol | 1 |
| Glycerin | 5 |
| 1,3-butylene glycol | 5 |
| Polyoxyethylene-hydrogenated castor oil succinate (50 E.O.) | 40 |
| Potassium hydroxide | 1 |
| 2-ethylhexyl paramethoxycinnamate | 0.1 |
| Purified water | Balance |
| Fragrance | As suitable |

### Example 3.15 - Hair Liquid

A hair liquid was prepared in accordance with ordinary methods containing a drug of the present invention according to the formula indicated below. The resulting hair liquid was a transparent solid and had a viscosity of 8. This hair liquid had a favorable feel during use and was observed to improve hair resiliency and body.

| | |
|---|---|
| 3-amino-5-methylisoxazole | 3 |
| Ethanol | 55 |
| Propylene glycol | 5 |
| Polyoxyethylene-polyoxypropylene-pentaerythritol ether (5 E.O.) | 25 |
| 2-ethylhexyl paramethoxycinnamate | 0.5 |
| Pigment | As suitable |
| Purified water | Balance |
| Fragrance | As suitable |

### Example 3.16 - Treatment

A treatment was prepared in accordance with ordinary methods containing a drug of the present invention according to the formula indicated below. The resulting treatment was an emulsified cream and had a viscosity of 25000. This treatment had a favorable feel during use and was observed to improve hair resiliency and body.

| | |
|---|---|
| 3-amino-5-methylisoxazole | 0.5 |
| Liquid paraffin | 10 |
| Vaseline | 3 |
| Dimethyl polysiloxane | 2 |
| Propylene glycol | 10 |
| Polyoxypropylene (40) butyl ether | 2 |
| Pentaerythritol tetra(2-ethylhexanoate) | 3 |
| Polyoxyethylene-hydrogenated castor oil | 2 |
| Potassium hydroxide | 0.1 |
| Carboxyvinyl polymer | 0.3 |
| Purified water | Balance |
| Fragrance | As suitable |

### Example 3.17 - Mousse

A mousse was prepared in accordance with ordinary methods containing a drug of the present invention according to the formula indicated below. The ratio of undiluted liquid to propellant was 90/10. The resulting mousse was a foam (aerosol) and had a viscosity of 5. This mousse had a favorable feel during use and was observed to improve hair resiliency and body.

| Stock: | |
|---|---|
| 3-amino-5-methylisoxazole | 0.5 |
| Volatile isoparaffin | 0.5 |
| Ethanol | 10 |
| Polyoxyethylene-hydrogenated castor oil | 0.5 |
| Polyoxyethylene-polyoxypropylene decyl ether | 0.5 |
| Loquat leaf extract | 0.1 |
| Yuka Foamer SM | 10 |
| Purified water | Balance |
| Propellant: | |
| LPG | |

### Example 3.18 - Hair Tonic

A hair tonic was prepared in accordance with ordinary methods containing a drug of the present invention according to the formula indicated below. The resulting hair tonic was a transparent liquid and had a viscosity of 7. This hair tonic had a favorable feel during use and was observed to improve hair resiliency and body.

| | |
|---|---|
| 3-amino-5-methylisoxazole | 3 |
| Ethanol | 60 |
| Dipropylene glycol | 2 |
| Polyoxyethylene-hydrogenated castor oil | 0.5 |
| Lactic acid | As suitable |
| Sodium lactate solution | As suitable |
| Monoammonium glycyrrhizate | 0.1 |
| Nicotinic amide | 0.1 |
| DL-α-tocopherol acetate | 0.1 |
| L-menthol | 0.2 |
| Pigment | As suitable |
| Purified water | Balance |
| Fragrance | As suitable |

### Example 3.19 - Hair Tonic

A hair tonic was prepared in accordance with ordinary methods containing a drug of the present invention according to the formula indicated below. The resulting hair tonic was a transparent liquid and had a viscosity of 7. This hair tonic had a favorable feel during use and was observed to improve hair resiliency and body.

| | |
|---|---|
| 3-acetamido-5-methylisoxazole | 2 |
| Ethanol | 60 |
| Dipropylene glycol | 2 |
| Polyoxyethylene-hydrogenated castor oil | 0.5 |
| Lactic acid | As suitable |
| Sodium lactate solution | As suitable |
| Monoammonium glycyrrhizate | 0.1 |
| Nicotinic amide | 0.1 |
| DL-α-tocopherol acetate | 0.1 |
| L-menthol | 0.2 |
| Pigment | As suitable |
| Purified water | Balance |
| Fragrance | As suitable |

### Example 3.20 - Hair Tonic

A hair tonic was prepared in accordance with ordinary methods containing drugs of the present invention according to the formula indicated below. The resulting hair tonic was a transparent liquid and had a viscosity of 7. This hair tonic had a favorable feel during use and was observed to improve hair resiliency and body.

| | |
|---|---|
| 3-amino-5-methylisoxazole | 1 |
| 3-acetamido-5-methylisoxazole | 1 |
| 2-acetamidothiazole | 1 |
| Ethanol | 60 |
| Dipropylene glycol | 2 |
| Polyoxyethylene-hydrogenated castor oil | 0.5 |
| Lactic acid | As suitable |
| Sodium lactate solution | As suitable |
| Monoammonium glycyrrhizate | 0.1 |
| Nicotinic amide | 0.1 |
| DL-α-tocopherol acetate | 0.1 |
| L-menthol | 0.2 |
| Pigment | As suitable |
| Purified water | Balance |
| Fragrance | As suitable |

### Example 3.21 - Hair Restoration Agent

A hair restoration agent was prepared in accordance with ordinary methods containing a drug of the present invention according to the formula indicated below. The resulting hair restoration agent was a transparent liquid and had a viscosity of 7. This hair restoration agent had a favorable feel during use and was observed to improve hair resiliency and body.

| | |
|---|---|
| 3-amino-5-methylisoxazole | 3 |
| Ethanol | 80 |
| Isostearyl alcohol | 2 |
| 1,3-butylene glycol | 3 |
| Polyoxyethylene-hydrogenated castor oil | 1 |
| Sodium lauryl sulfate | 0.3 |
| DL-malic acid | As suitable |
| β-glycyrrhetinic acid | 0.2 |
| Pantotenyl ethyl ether | 0.1 |
| Benzyl nicotinate | 0.1 |
| Nicotinic amide | 0.1 |
| DL-α-tocopherol acetate | 0.5 |
| Decyltetradecyl dimethylamine oxide solution (20%) | 5 |
| L-menthol | 1 |

### Example 3.22 - Hair Restoration Agent

A hair restoration agent was prepared in accordance with ordinary methods containing a drug of the present invention according to the formula indicated below. The resulting hair restoration agent was a transparent liquid and had a viscosity of 7. This hair restoration agent had a favorable feel during use and was observed to improve hair resiliency and body.

| | |
|---|---|
| 3-acetamido-5-methylisoxazole | 2 |
| Ethanol | 80 |
| Isostearyl alcohol | 2 |
| 1,3-butylene glycol | 3 |
| Polyoxyethylene-hydrogenated castor oil | 1 |
| Sodium lauryl sulfate | 0.3 |
| DL-malic acid | As suitable |
| β-glycyrrhetinic acid | 0.2 |
| Pantotenyl ethyl ether | 0.1 |
| Benzyl nicotinate | 0.1 |
| Nicotinic amide | 0.1 |
| DL-α-tocopherol acetate | 0.5 |
| Decyltetradecyl dimethylamine oxide solution (20%) | 5 |
| L-menthol | 1 |

### Example 3.23 - Hair Restoration Agent

A hair restoration agent was prepared in accordance with ordinary methods containing a drug of the present invention according to the formula indicated below. The resulting hair restoration agent was a transparent liquid and had a viscosity of 7. This hair restoration agent had a favorable feel during use and was observed to improve hair resiliency and body.

| | |
|---|---|
| 3-acetamido-5-methylisoxazole | 2 |
| Ethanol | 80 |
| Isostearyl alcohol | 2 |
| 1,3-butylene glycol | 3 |
| Polyoxyethylene-hydrogenated castor oil | 1 |
| Sodium lauryl sulfate | 0.3 |
| DL-malic acid | As suitable |
| β-glycyrrhetinic acid | 0.2 |
| Pantotenyl ethyl ether | 0.1 |
| Benzyl nicotinate | 0.1 |
| Nicotinic amide | 0.1 |
| DL-α-tocopherol acetate | 0.5 |
| Decyltetradecyl dimethylamine oxide solution (20%) | 5 |
| L-menthol | 1 |

### Example 3.24 - Hair Lotion

A hair lotion was prepared in accordance with ordinary methods containing a drug of the present invention according to the formula indicated below. The resulting hair lotion was a transparent liquid and had a viscosity of 3000. This hair lotion had a favorable feel during use and was observed to improve hair resiliency and body.

| | |
|---|---|
| 2-acetamido-4-methylthiazole | 0.1 |
| Ethanol | 50 |
| Dipropylene glycol | 5 |
| Polyoxyethylene-hydrogenated castor oil | 0.5 |
| Malic acid | As suitable |
| Monoammonium glycyrrhizate | 0.1 |
| Nicotinic amide | 0.1 |
| DL-α-tocopherol acetate | 0.1 |
| L-menthol | 0.2 |
| Crosslinked N,N-dimethylacrylamide/sodium 2-acrylamido-2-methylpropane sulfonate copolymer | 0.3 |
| Pigment | As suitable |
| Purified water | Balance |
| Fragrance | As suitable |

### INDUSTRIAL APPLICABILITY

A hair resiliency and body improver and hair cosmetic of the present invention are able to improve hair resiliency and body by penetrating into the hair or being absorbed into hair follicles when used on the hair or scalp, thereby making it useful as a beauty regimen.

## Claims

1. A hair resiliency and body improver comprising as the active ingredient one or more types of compounds selected from the group consisting of 2-aminothiazole derivatives represented by the following general formula (1) and pharmaceutically acceptable salts thereof: 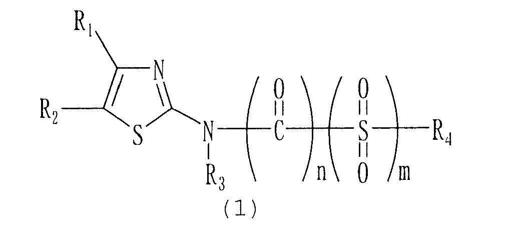 wherein, R₁, R₂, R₃ and R₄ independently represent a hydrogen atom, linear or branched alkyl group having 1 to 8 carbon atoms, linear or branched alkenyl group having 2 to 8 carbon atoms, cycloalkyl group having 3 to 8 carbon atoms, aralkyl group having 7 to 10 carbon atoms, aryl group having 6 to 14 carbon atoms or heteroaryl group having 5 to 14 members, and n and m represent 0 or 1, provided that when n is 1, m is 0 and R₃ is a hydrogen atom or methyl group, and when m is 1, n is 0 and R₃ is a hydrogen atom or methyl group.

2. The hair resiliency and body improver according to claim 1, wherein R₂ in the general formula (I) is a hydrogen atom.

3. The hair resiliency and body improver according to either claim 1 or 2, wherein the 2-aminothiazole derivative is selected from the group consisting of 2-aminothiazole, 2-acetamidothiazole and 2-acetamido-4-methylthiazole.

4. A hair cosmetic comprising as the active ingredient one or more types of compounds selected from the group consisting of 2-aminothiazole derivatives represented by the following general formula (1) and pharmaceutically acceptable salts thereof: 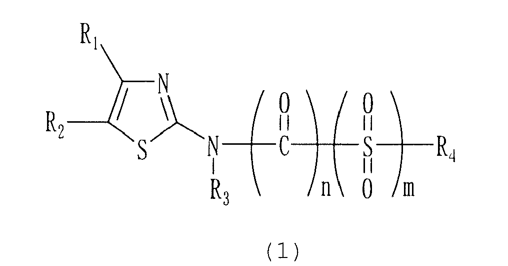 wherein, R₁, R₃ and R₄ independently represent a hydrogen atom, linear or branched alkyl group having 1 to 8 carbon atoms, linear or branched alkenyl group having 2 to 8 carbon atoms, cycloalkyl group having 3 to 8 carbon atoms, aralkyl group having 7 to 10 carbon atoms, aryl group having 6 to 14 carbon atoms or heteroaryl group having 5 to 14 members, R₂ represents a hydrogen atom, and n and m represent 0 or 1, provided that when n is 1, m is 0 and R₃ is a hydrogen atom or methyl group, and when m is 1, n is 0 and R₃ is a hydrogen atom or methyl group.

5. The hair cosmetic according to claim 4, wherein the 2-aminothiazole derivative is selected from the group consisting of 2-aminothiazole, 2-acetamidothiazole and 2-acetamido-4-methylthiazole.

6. A method for improving hair resiliency and body comprising the step of applying one or more types of compounds selected from the group consisting of the 2-aminothiazole derivatives and pharmaceutically acceptable salts thereof according to claim 1, to the hair or scalp.

7. A method for preparing a hair cosmetic comprising the step of producing a hair cosmetic by adding one or more types of compounds selected from the group consisting of the 2-aminothiazole derivatives and pharmaceutically acceptable salts thereof according to claim 4, to an aqueous phase or oily phase.

8. A hair cosmetic comprising as the active ingredient one or more types of compounds selected from the group consisting of 3-aminoisoxazole derivatives represented by the following general formula (2) and pharmaceutically acceptable salts thereof: 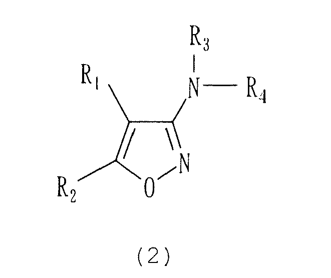 wherein, R₁ represents a hydrogen atom or methyl group, R₂ represents a hydrogen atom, linear or branched alkyl group having 1 to 8 carbon atoms, linear or branched alkenyl group having 2 to 8 carbon atoms, cycloalkyl group having 3 to 8 carbon atoms, aralkyl group having 7 to 10 carbon atoms or aryl group having 6 to 14 carbon atoms, R₃ represents a hydrogen atom or methyl group, and R₄ represents a hydrogen atom, linear or branched alkyl group having 1 to 8 carbon atoms, linear or branched alkenyl group having 2 to 8 carbon atoms, cycloalkyl group having 3 to 8 carbon atoms, aralkyl group having 7 to 10 carbon atoms, aryl group having 6 to 14 carbon atoms, alkylcarbonyl group having 2 to 8 carbon atoms, or arylcarbonyl group having 7 to 10 carbon atoms, and the aryl moiety of the aralkyl groups, aryl groups and arylcarbonyl groups may be independently substituted with an alkyl group having 1 to 3 carbon atoms, an alkoxy group having 1 to 3 carbon atoms or a hydroxyl group).

9. A hair resiliency and body improver comprising as the active ingredient one or more types of compounds selected from the group consisting of the 3-aminoisoxazole derivatives of the general formula (2) and pharmaceutically acceptable salts thereof according to claim 8.
